# EUROPÄISCHE PATENTSCHRIFT

(11) **EP 2 925 258 B1**
(45) Veröffentlichungstag und Bekanntmachung des Hinweises auf die Patenterteilung: **09.10.2019**
(21) Anmeldenummer: 13795250.3
(22) Anmeldetag: 25.11.2013
(51) Int. Cl.: A61F 2/30, A61F 2/46, A61L 27/10, A61F 2/44, A61L 27/56, C04B 35/48, C04B 35/10, A61L 27/54

(54) **ENDOPROTHETISCHES BAUTEIL**
ENDOPROSTHETIC COMPONENT
ÉLÉMENT ENDOPROTHÉTIQUE

(30) Priorität: 26.11.2012 DE 102012221523
(43) Veröffentlichungstag der Anmeldung: 07.10.2015
(73) Patentinhaber: CeramTec GmbH, 73207 Plochingen (DE)
(72) Erfinder: KELNBERGER, Alfons, 90552 Röthenbach (DE); WECKER, Heinrich, 90542 Eckental (DE); ZIERMANN, Frank, 10967 Berlin (DE); JUSZCZYK, Mateusz, 91235 Velden (DE)
(74) Vertreter: Fehrenbacher, Eckhard Anton
(86) Internationale Anmeldenummer: PCT/EP2013/074576
(87) Internationale Veröffentlichungsnummer: WO 2014/080008

(56) Entgegenhaltungen:
- WO-A1-2012/063865
- US-A1- 2003 105 527
- US-A1- 2009 157 187
- US-A1- 2010 042 216
- US-A1- 2011 012 280

## Beschreibung

Die Erfindung betrifft endoprothetische Bauteile. Insbesondere betrifft die Erfindung endoprothetische Bauteile, die als Wirbelsäulen-Implantate Verwendung finden können.

Endoprothetische Bauteile, insbesondere zur Fusionierung von Wirbelkörpern, sind bekannt. Sie sind in ihrer Geometrie der Anatomie des menschlichen Wirbelkörpers angepasst, befinden sich zwischen zwei Wirbelköpern und ersetzen die Bandscheibe ganz oder teilweise.

Typischerweise halten sie in einer ersten Phase des Verbleibs im menschlichen Körper allein durch ihre mechanischen Eigenschaften die Wirbelkörper auf Distanz und in einer anatomisch korrekten Position. Sie fördern die Fusionierung und damit das Verwachsen der beiden sie umgebenden Wirbelkörper in einer zweiten Phase.

Bekannte Bauteile zur Fusionierung von Wirbelkörpern basieren beispielsweise auf metallischen Werkstoffen wie z.B. Tantal oder Titan.

Nachteile dieser metallischen Werkstoffe sind beispielsweise:
- Metallischer Abrieb und daraus resultierende negative Auswirkungen auf den menschlichen Organismus
- Artefakte in der Bildgebung bei der medizinischen Diagnostik
- Alterungseffekte und Langzeitverhalten wie Ermüdung, Korrosion und Freisetzung von metallischen Ionen, die toxisch wirken können

Als generelles Problem stellt sich mehr und mehr ein Infektionsrisiko während der Operation heraus, das mit keramischen Bauteilen reduziert werden kann.

Auch Bauteile basierend auf Kunststoffen wie z.B. hochvernetzte PE- (Polyethylen) Werkstoffe oder PEEK (Polyethylenetherketon) sind bekannt.

Nachteile der Kunststoffbauteile sind z.B.:
- Unzureichende mechanische Eigenschaften wie z.B. das Abbrechen von Zacken oder sonstigen Bestandteilen des Bauteils beispielsweise beim Einbau.
- Mangelnde Darstellbarkeit bei den gängigen Bildgebungsverfahren (MRI, Röntgen), dadurch bedingter Einsatz von metallischen Markern.
- Alterungseffekte und Langzeitverhalten, insbesondere Ermüdung des Materials.

Bekannt sind auch keramische Bauteile beispielsweise basierend auf Siliziumnitrid. Diese Werkstoffklasse wurde jedoch mit Blick auf exzellente Hochtemperatureigenschaften entwickelt - beispielsweise zur mechanischen Bearbeitung von metallischen Bauteilen für die Automobilindustrie - und rangiert bei den für diese Anwendung geforderten Eigenschaften wie Festigkeit, Härte und Langzeitstabilität im Vergleich zu anderen keramischen Hochleistungswerkstoffen basierend auf oxidischen Systemen eher im Mittelfeld.

Außerdem handelt es sich um einen relativ komplizierten Werkstoff, bei dem nadelförmiges Siliziumnitrid in eine Glasmatrix eingebettet ist. Entsprechend aufwändig ist die Sinterung dieses Werkstoffs. Die mechanische (Nach-)Bearbeitung, beispielsweise mittels Schleifens oder Polierens, ist extrem anspruchsvoll und schwierig.

Darüber hinaus weisen aus Siliziumnitrid gefertigte Bauteile eine eher dunkle Färbung - grau bis schwarz - auf, was aus rein optischen Gründen im medizinischen Bereich auf eine geringe Akzeptanz stößt.

Alle diese Nachteile führen zu erhöhten Kosten bei der Herstellung der Bauteile, was einen weiteren Nachteil darstellt.

Ein ganz wichtiger Aspekt bei der Verwendung von keramischen Bauteilen zur Fusionierung von Wirbelkörpern ist die generell hohe Steifigkeit dieser Werkstoffe, die wesentlich höher ist als die der menschlichen Wirbelkörper.

Dies kann unter Umständen zu dem Effekt des sog. "stress shielding" führen, was den Abbau von Knochenmaterial nach sich ziehen kann und den Aufbau von neuem Knochenmaterial zumindest reduziert oder sogar unterbindet. Eine Fusion von Wirbelkörpern findet dann nicht statt.

Das Prinzip dieses Effekts lässt sich wie folgt etwas genauer erklären: Bekannte keramische Cages sind in der Regel ringförmig ausgeführt bzw. der Form menschlicher Wirbelkörper angepasst, wobei der Ring aus einer monolithischen, also dichten, festen und sehr steifen Keramik besteht. Im Zentrum weisen diese Cages häufig einen Hohlraum auf, der entweder mit bekannten Knochenersatzmaterialien (autolog oder allogen) aufgefüllt wird oder eine künstliche, poröse, osseoinduktive oder osseokonduktive Struktur aufweist, die in der Regel wesentlich weniger steif ist als der äußere Ring. In diesem Bereich sollen die Knochenzellen neues Knochenmaterial aufbauen, wobei die daran beteiligten Zellen einen entsprechenden mechanischen Stimulus benötigen.

Werden nun die Kräfte, die durch biomechanische Belastungen des Bauteils entstehen, durch den Bereich mit der hohen Steifigkeit geleitet, fehlt der mechanische Stimulus in dem zentralen Bereich des Bauteils - also dort, wo die Fusion erfolgen soll - weil die Belastung abgeschirmt wird ("stress shielding"). Aufgrund des in diesem Bereich fehlenden mechanischen Stimulus, findet kein Knochenaufbau und damit auch keine Fusion statt.

Dieser ganz entscheidende Nachteil soll durch die vorliegende Erfindung gelöst werden.

Ein weiterer wesentlicher Nachteil der bekannten Lösungen ist das unkontrollierte Einsinken der Bauteile in die Knochenstruktur der Wirbelkörper. Dieses unkontrollierte Einsinken kann auftreten, wenn bedingt durch die Geometrie des Bauteils eine hohe punktförmige Belastung auf eine relativ weiche Knochensubstanz einwirkt.

Die Knochenstruktur eines Wirbelkörpers ist unterschiedlich, beispielsweise ist die äußere kortikale Knochensubstanz wesentlich dichter und fester als die innere spongiöse Knochensubstanz. Darüber hinaus ist die Knochenstruktur eines Menschen von seinem Alter abhängig und natürlich auch individuell verschieden. Je nach Gewicht und Aktivität des Menschen wirken unterschiedliche biomechanische Belastungen auf die Wirbelsäule. Infolge dessen kann es zum unkontrollierten Einsinken der Bauteile in die Wirbelkörper kommen, was unterschiedlichste Komplikationen und Folgen haben kann.

Dieser Nachteil soll durch die vorliegende Erfindung gelöst werden.

Demgemäß besteht die Aufgabe der Erfindung in der Bereitstellung eines endoprothetischen Bauteils, das insbesondere für die Fusionierung von Wirbelkörpern geeignet ist und den Effekt des "stress shielding" vermeidet oder zumindest vermindert.

Dabei sind folgende Aufgaben, einzeln oder in Kombination, wesentlich:
- Das Bauteil sollte eine ausreichend hohe Festigkeit aufweisen, damit die Wirbelkörper während der Fusion in einer definierten Position zueinander gehalten werden
- Das Bauteil sollte eine ausreichend hohe mechanische Stimulation zur Bildung von neuem Knochenmaterial gewährleisten. Es soll "stress shielding" vermieden werden, damit die Fusion stattfinden kann.
- Das Bauteil sollte eine ausreichend hohe Primärstabilität gewährleisten, d.h. es sollte während der Fusion stabil zwischen den Wirbelkörpern verbleiben.
- Das Bauteil sollte nicht unkontrolliert in die Knochensubstanz der Wirbelkörper einsinken
- Das Bauteil sollte die Fusion zweier Wirbelkörper möglichst rasch und komplett sicherstellen.
- Das Bauteil sollte nach erfolgter Fusion in hohem Maß bioinert sein, d.h. es sollten keine biologischen Wechselwirkungen zwischen dem Bauteil und dem umgebenden Gewebe stattfinden. Insbesondere sollen auch keine schädlichen Abriebpartikel entstehen, die über das Gefäßsystem im menschlichen Körper verteilt werden und unkontrollierbare Reaktionen nach sich ziehen können.

Die Aufgabe wird mit einem endoprothetischen Bauteil mit den Merkmalen des Anspruchs 1 gelöst. Vorteilhafte Ausgestaltungen dieses Gegenstands sind in den Unteransprüchen normiert.

Ein erfindungsgemäßes endoprothetisches Bauteil, umfasst einen ersten Bereich und einen zweiten Bereich, wobei das Material des ersten Bereichs eine größere Festigkeit als das Material des zweiten Bereichs aufweist. Der erste Bereich ist dazu eingerichtet, im implantierten Zustand in angrenzendes Knochenmaterial kontrolliert einzudringen.

Besonders bevorzugt ist das endoprothetische Bauteil ein Wirbelsäulen-Implantat, insbesondere ein Cage. Anders ausgedrückt, wird die Verwendung eines solchen endoprothetischen Bauteils als Wirbelsäulen-Implantat, insbesondere als Cage bevorzugt.

Unter kontrolliertem Eindringen soll im Rahmen dieser Erfindung verstanden werden, dass nur zum Eindringen in den Knochen/Wirbel vorgesehene Teile des Bauteils mit einer vorbestimmten maximalen Tiefe in den Knochen/Wirbel eindringen. Ein unkontrolliertes Einsinken von nicht zum Eindringen vorgesehenen Teilen des Bauteils in den Knochen/Wirbel, soll von diesem Begriff nicht erfasst sein.

Vorteilhaft kann dies dadurch erreicht werden, dass zumindest ein Teil einer Oberfläche des ersten Bereichs über eine Oberfläche des zweiten Bereichs hinausragt. Der über die Oberfläche des zweiten Bereichs hinausragende Teil des ersten Bereichs kann dann so gestaltet werden, dass er kontrolliert in die angrenzenden Wirbelkörper eindringen kann. Eine mögliche Ausführungsform könnte in Spitzen oder Dornen bestehen, die über die Oberfläche des zweiten Bereichs hinausragen und sich im implantierten Zustand in die Oberfläche des angrenzenden Wirbelkörpers bohren. Über die Länge und den Durchmesser der Dornen kann eine maximale Eindringtiefe definiert werden, so dass das Eindringen kontrolliert ist.

Gemäß einer bevorzugten Ausführungsform der Erfindung umschließt der erste äußere Bereich des Bauteils den zweiten inneren Bereich teilweise oder vollständig.

Der erste Bereich besteht bevorzugt aus dicht gesinterter, monolithischer Keramik. Das heißt, dass der erste Bereich im Wesentlichen nicht porös ist, also bevorzugt eine Porosität kleiner 5 Vol.-%, besonders bevorzugt eine Porosität kleiner 3 Vol.-% und insbesondere bevorzugt eine Porosität kleiner 1 Vol.-% aufweist.

Als Material kommen bevorzugt oxidische Werkstoffklassen, wie z.B. die Klasse der Aluminiumoxide oder Zirkonoxide oder beliebigen Mischungen davon zum Einsatz.

Die Aluminiumoxide umfassen erfindungsgemäß auch Zirkonoxid-verstärkte Werkstoffe wie z.B. ZTA-Werkstoffe (zirconia thoughened alumina); unter die Zirkonoxide fallen alle Arten von tetragonal stabilisierten oder teilstabilisierten Zirkonoxiden, wie z.B. Yttrium-, Cer- oder Gadolinium-stabilisierte Zirkonoxide. Es sind auch zirkonoxidbasierte Verbundwerkstoffe mit aluminiumoxidhaltigen Anteilen zur Verstärkung denkbar, sog. ATZ Werkstoffe.

Da diese Werkstoffe hohe Festigkeiten aufweisen, ist sichergestellt, dass das Bauteil insgesamt eine ausreichend und hohe Festigkeit besitzt. Die standardmäßig gemessenen 4-Punkt-Biegefestigkeiten dieser Werkstoffe liegen im Bereich zwischen ca. 500 bis knapp 2000 MPa.

Der zweite innere Bereich besteht aus einer porösen Keramik, wobei es sich hierbei prinzipiell um das gleiche oder ein ähnliches Material wie das des ersten Bereichs handeln kann, aber nicht muss. Gemäß einer bevorzugten Ausführungsform kann das Material des inneren ersten und/oder des äußeren zweiten Bereichs durch zusätzliche Beigaben bioaktiver Substanzen bioaktiviert sein. Dafür in Frage kommen beispielsweise Schichten, Bereiche, Beimischungen von bekannten bioaktiven Substanzen in das Material aus dem der erste und/oder der zweite Bereich gefertigt werden oder auch Beschichtungen von zumindest Teilbereichen der Oberflächen dieser Bereiche. Die bioaktiven Substanzen können aber auch in den Poren des zweiten Bereichs angeordnet sein.

Besonders geeignet sind Substanzen basierend auf Calciumphosphat, wie z.B. Hydroxylapatit (HA) oder Tricalciumphosphat (TCP), jegliche Formen von modifizierten HA-Schichten, bei denen die Ca-Atome ersetzt sind durch Elemente der Alkali- oder Erdalkaligruppe wie Na, K, oder Mg, Sr, aber auch beispielsweise Si.

Besonders geeignet sind auch glasähnliche Substanzen wie z.B. Biogläser. Vorzugsweise wird ein Bioglas, das als Hauptbestandteile SiO₂ (Siliziumdioxid), CaO (Calciumoxid), Na₂O (Natriumhyperoxid) und P₂O₅ (Phosphorpentoxid) umfasst, verwendet.

Zur Biofunktionalisierung im Sinne der Förderung und Stimulation des Knochenwachstums können auch alle Arten von Beschichtungen verwendet werden, die sich des biomimetischen Ansatzes bedienen und so den an der Knochenbildung beteiligten Zellen optimale Bedingungen zur Osteogenese bieten. Dies können nanostrukturierte HA Beschichtungen oder kovalent gebundene Phosphatierungsschichten sein.

Für den Fachmann klar ersichtlich, können der erste und/oder der zweite Bereich des endoprothetischen Bauteils natürlich auch aus anderen als den aufgezählten Keramiken, insbesondere aus Siliziumnitrid oder überhaupt anderen Materialien, z.B. aus Kunststoffen wie PE, PEEK oder gewebeverträglichen Metallen wie Titan hergestellt sein, solange diese Materialien die genannten Anforderungen erfüllen.

Die innere Struktur des zweiten inneren Bereiches hat ganz entscheidenden Einfluss auf die Osseokonduktivität des Bauteils, also auf die Fähigkeit neues Knochenmaterial aufzubauen. Poröse, trabekuläre Strukturen sind besonders geeignet, um die Knochenneubildung anzuregen. Diese Strukturen sind der Struktur von spongiösem Knochen sehr ähnlich und sorgen für optimale Bedingungen zum Wachstum der am Knochenaufbau beteiligten Zellen.

Entscheidend sind dabei folgende Parameter:
- Porengrößen zwischen 100 und 1000 µm, bevorzugt zwischen 400 und 600 µm
- Porositäten zwischen 75 und 85 Vol.-%
- Interkonnektivität, d.h. die einzelnen Poren sind miteinander verbunden und ermöglichen eine optimale Vaskularisierung des Bauteils

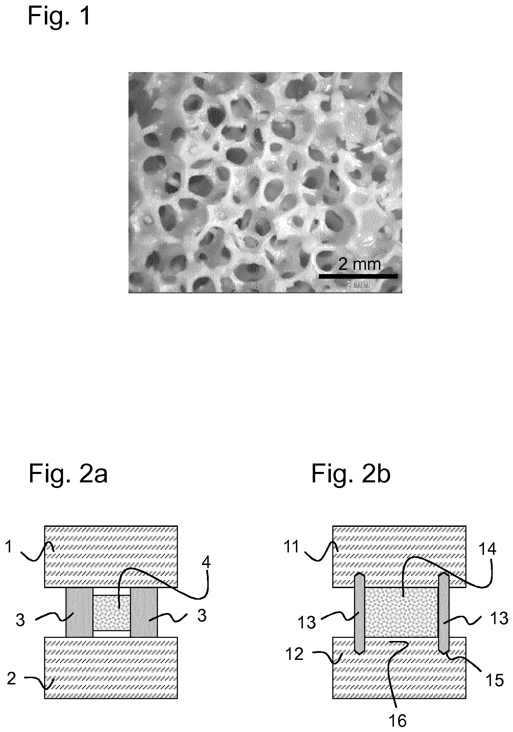

Eine mögliche Umsetzung dieser Vorgaben in keramisches Material zeigt Fig. 1. Hier ist in einer ZTA-Keramik eine interkonnektive Porosität mit der bevorzugten Porengröße von 400 bis 600 µm verwirklicht.

Die mechanischen Eigenschaften des zweiten inneren Bereiches hängen sehr stark von der Materialzusammensetzung und von der Struktur ab. Dies bedeutet allerdings auch, dass über die Auswahl des Materials und das Design der Strukturen die mechanischen Eigenschaften des inneren Bereichs gezielt eingestellt und auf das biologische Umfeld, in das sie eingebracht werden sollen, angepasst werden können. In der Regel ist der zweite, trabekuläre Bereich des Bauteils weniger fest und weniger steif als der erste, äußere Bereich des Bauteils. Denkbar sind jedoch auch Strukturen, bei denen der erste steife Bereich im Inneren des Bauteils, beispielsweise als Pfeiler, vorgesehen ist und der zweite tabekuläre Bereich um diesen Pfeiler herum angeordnet ist.

Die tatsächliche Anordnung dieser beiden Bereiche ist grundsätzlich nicht limitiert. Wesentlich ist nur das Vorhandensein der beiden Bereiche und die Erfüllung der Aufgaben in dem Bauteil.

Druckfestigkeiten für solche trabekulären Keramikstrukturen liegen typischerweise im Bereich von 5 bis 10 MPa. Durch die Verwendung geeigneter Herstellungsverfahren können jedoch auch Druckfestigkeiten bis in den oberen zweistelligen Megapascal Bereich erreicht werden. Die entsprechenden Steifigkeiten je nach Geometrie liegen im Bereich von 5 bis 50 GPa.

Entscheidend für das erfindungsgemäße Bauteil ist die Kombination dieser beiden Bereiche bzw. das Design des durch die Kombination entstehenden Bauteils.

Ist der erste äußere Bereich zu massiv, dann entsteht aufgrund der hohen Steifigkeit des monolithischen Keramikmaterials der Effekt des schon beschriebenen "stress shielding". Dadurch verliert das Bauteil insgesamt seine Fusionsfunktion. Der zweite, innere Bereich wird von der Belastung abgeschirmt und aufgrund der fehlenden mechanischen Stimulation findet kein Knochenaufbau statt.

Allerding ermöglicht ein massiver äußerer Bereich eine große Auflagefläche des mechanisch belastbaren ersten Bereichs des Bauteils auf den Wirbelkörpern, wodurch einem Einsinken des Bauteils entgegengewirkt wird.

Minimiert man die strukturelle Steifigkeit zur Vermeidung von "stress shielding" durch Reduktion des ersten äußeren Bereichs im Verhältnis zum zweiten inneren Bereich und führt diesen dann zu schwach aus, beispielsweise durch eine zu dünne Wandstärke, so kann das Bauteil unkontrolliert in die benachbarten Wirbelkörper einsinken.

Eine geringe Wandstärke des ersten äußeren Bereichs kann auch zu einer insgesamt zu geringen mechanischen Stabilität des Bauteils führen, so dass die Gefahr des Bruchs des Bauteils, insbesondere während der Fusionsphase, besteht.

Gemäß einer besonders bevorzugten Ausführungsform der Erfindung wird die Auflagefläche des ersten Bereichs soweit reduziert, dass die Festigkeit des Bauteils noch ausreichend hoch ist, um die mechanische Stabilität des Bauteils insgesamt zu gewährleisten. Damit nimmt die strukturelle Steifigkeit des Bauteils insgesamt ab und nähert sich der eines Wirbelkörpers an. Dies vermindert die Gefahr des "stress shieldings".

Typische Wandstärken für den ersten Bereich liegen im Bereich von einigen Millimetern, je nachdem ob es sich um ein Bauteil für den lumbalen oder cervicalen Bereich handelt. Typische Werte für cervicale Bauteile liegen zwischen 2 und 3 mm, für lumbale Bauteile zwischen 4 und 6 mm.

Zur Vermeidung des unkontrollierten Einsinkens ("subsidence") des Implantats in den Wirbelkörper weist der erste Bereich an den Auflageflächen Strukturen auf, beispielsweise eine umlaufende Spitze oder Zacken, die ein definiertes Eindringen des Bauteils in die Wirbelkörper ermöglichen und begünstigen.

Im Idealfall dringen die Strukturen des ersten Bereiches soweit in die Wirbelkörper ein, bis der poröse zweite Bereich flächig auf den beiden angrenzenden Wirbelkörpern aufliegt.

Dieser Idealfall kann sowohl im Rahmen des operativen Eingriffs erzwungen als auch durch weitere konstruktive Details des Bauteils begünstigt werden.

Daher sieht ein bevorzugte Ausführungsform der Erfindung vor, dass der erste Bereich dazu eingerichtet ist, so tief in das angrenzende Knochenmaterial einzudringen, dass zumindest ein Teil der Oberfläche des zweiten Bereichs in Kontakt mit dem Knochenmaterial steht, so dass eine Kraftübertragung zwischen Knochenmaterial und der Oberfläche des zweiten Bereichs stattfindet. Diese Ausführungsform hat eben den Vorteil, dass es Design-bedingt nicht zu einer Kraftabschirmung des zweiten Bereichs kommen kann und daher optimale Bedingungen für die Stimulation des Knochenaufbaus in diesem Bereich geschaffen werden.

Ein schematisches Beispiel für eine solche Ausführungsform wird in Fig. 2 gezeigt. Figur 2a zeigt einen herkömmlichen Wirbelsäulen-Cage, der zwischen zwei Wirbeln 1 und 2 angeordnet ist. Der erste äußere Bereich 3 ist ringförmig ausgeführt und umschließt den zweiten inneren Bereich 4. Der innere Bereich 4 weist eine geringere Höhe auf als der äußeren Ring 3 und kommt so nicht in Kontakt mit den Oberflächen der angrenzenden Wirbel. Bei einem solchen Design kommt es zum beschriebenen "stress shielding". Darüber hinaus besteht die Gefahr des unkontrollierten Einsinkens des Bauteils in die Oberflächen der angrenzenden Wirbelkörper.

Figur 2b zeigt eine mögliche Ausführungsform der Erfindung. Auch hier besteht der erste äußere Bereich aus einem monolithischen Keramikring 13, der eine poröse Keramikfüllung, den zweiten inneren Bereich 14, umschließt. Im Gegensatz zu dem herkömmlichen Bauteil aus Fig.2a ist der Keramikring 13 jedoch deutlich weniger stark ausgeführt und ragt über den zweiten inneren Bereich 14 oben und unten hinaus. Der obere und der untere Rand 15 des Keramikrings 13 ist spitz zulaufend geformt, so dass diese Bereiche leicht in die Oberflächen 16 der angrenzenden Wirbelkörper 11 und 12 eindringen können. Natürlich sind auch andere Elemente möglich, die das Eindringen ermöglichen, beispielsweise isolierte Spitzen. Wesentlich ist, dass das Eindringen kontrolliert stattfindet. In der in Fig. 2b gezeigten Ausführungsform ist dies sichergestellt, indem zum einen nur die Teile, die für das Eindringen vorgesehen sind, auch eindringen können. Zum anderen wird die maximale Eindringtiefe durch die poröse Füllung des inneren zweiten Bereichs begrenzt. Durch dieses Design wird also gleichzeitig sichergestellt, dass der zweite innere Bereich mit den Oberflächen der angrenzenden Wirbelkörper in Kontakt kommt und eine mechanische Stimulation auf diese ausübt. Damit sind die Gefahren des "stress shieldings" und des unkontrollierten Einsinkens in die angrenzenden Wirbelknochen wirksam minimiert.

Ein weiteres bevorzugtes Merkmal betrifft die Verbindung des ersten Bereichs mit dem zweiten Bereich.

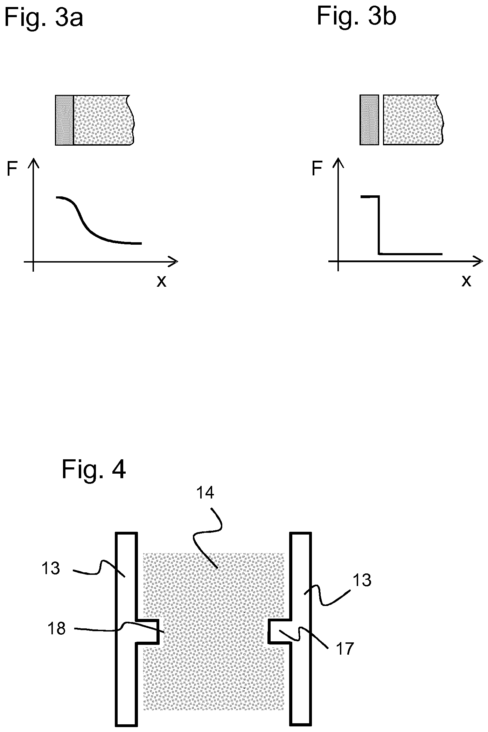

Je nachdem ob die beiden Bereiche fest oder beweglich miteinander verbunden sind, ergibt sich eine andere Lastverteilung. Im ersten Fall erfolgt ein stetiger Lastübergang zwischen den beiden Bereichen, im zweiten Fall ein unstetiger. Figur 3a zeigt den Kraftverlauf im Querschnitt eines Cages mit einer festen Verbindung zwischen dem ersten und dem zweiten Bereich. Die Kurve ist stetig. Figur 3b zeigt den Kraftverlauf im Querschnitt eines Cages mit einer beweglichen Verbindung. Die Kurve ist unstetig.

Günstiger für die Fusion ist ein unstetiger Lastübergang, weil die unterschiedlichen Steifigkeiten voneinander entkoppelt eingestellt werden können. Insbesondere kann der trabekuläre zweite Bereich aufgrund seiner Struktur und Materialzusammensetzung an die Steifigkeit des Wirbelkörpers ideal angepasst werden. Die mikromechanischen Bewegungen und damit der mechanischen Anreiz für die Knochenneubildung können so optimal und unabhängig vom ersten Bereich, der für die mechanische Stabilität und die Primärstabilität sorgt, eingestellt werden. Daher sieht eine besonders bevorzugte Ausführungsform vor, dass der erste Bereich beweglich mit dem zweiten Bereich verbunden ist.

Die Ausgestaltung der beweglichen Verbindung im Detail ist offen. Entscheidend ist, dass der trabekuläre zweite Bereich nach der Implantation flächig auf den angrenzenden Wirbelkörpern aufliegt und der erste monolithische Bereich definiert und gleichmäßig in die Wirbelkörper eindringen kann.

Figur 4 zeigt eine mögliche Ausführungsform, in der der ringförmige oder quadratische äußere erste Bereich 13 mit dem inneren zweiten Bereich 14 beweglich verbunden ist. Mittels einer umlaufenden Leiste 17 an der inneren Wand des äußeren ersten Bereichs 13 und einer dazu passenden Ausnehmung 18 in der Seitenwand des inneren zweiten Bereichs wird die poröse Füllung des zweiten Bereichs im monolithischen Ring des ersten Bereichs 13 beweglich gehalten.

## Patentansprüche

1. Endoprothetisches Bauteil, wobei das Bauteil einen ersten äußeren Bereich (13) und einen zweiten inneren Bereich (14) umfasst, wobei das Material des ersten Bereichs (13) eine größere Festigkeit als das Material des zweiten Bereich (14) aufweist, wobei der erste äußere Bereich des Bauteils den zweiten inneren Bereich teilweise umschließt wobei der erste Bereich (13) dazu eingerichtet ist, im implantierten Zustand in angrenzendes Knochenmaterial (11, 12) kontrolliert einzudringen, **dadurch gekennzeichnet, dass** der zweite Bereich (14) Poren mit Porengrößen zwischen 100 und 1000 µm, wobei die Porosität des zweiten Bereichs (14) interkonnektierend ist, und wobei zumindest ein Teil der Oberfläche des ersten Bereichs (13) über die Oberfläche des zweiten Bereichs (14) hinausragt.

2. Endoprothetisches Bauteil Anspruch 1, **dadurch gekennzeichnet, dass** der erste Bereich (13) dazu eingerichtet ist, so tief in das angrenzende Knochenmaterial (11, 12) einzudringen, dass zumindest ein Teil der Oberfläche des zweiten Bereichs (14) in Kontakt mit dem Knochenmaterial (11, 12) steht, so dass eine Kraftübertragung zwischen Knochenmaterial (11, 12) und der Oberfläche des zweiten Bereichs (14) stattfindet.

3. Endoprothetisches Bauteil nach einem der vorstehenden Ansprüche, **dadurch gekennzeichnet, dass** der erste Bereich (13) beweglich mit dem zweiten Bereich (14) verbunden ist.

4. Endoprothetisches Bauteil nach einem der vorstehenden Ansprüche, **dadurch gekennzeichnet, dass** der erste Bereich (13) nicht porös ist, das heißt bevorzugt eine Porosität kleiner 5 Vol.-% aufweist.

5. Endoprothetisches Bauteil nach einem der vorstehenden Ansprüche, **dadurch gekennzeichnet, dass** der zweite Bereich (14) porös ist, wobei die Porosität zwischen 75 und 85 Vol.-% liegt.

6. Endoprothetisches Bauteil nach dem vorstehenden Anspruch, **dadurch gekennzeichnet, dass** der zweite Bereich (14) Poren mit Porengrößen zwischen 400 und 600 µm aufweist.

7. Endoprothetisches Bauteil nach einem der vorstehenden Ansprüche, **dadurch gekennzeichnet, dass** der erste und/oder der zweite Bereich (13, 14) keramisches Material umfasst.

8. Endoprothetisches Bauteil nach einem der vorstehenden Ansprüche, **dadurch gekennzeichnet, dass** der erste und/oder der zweite Bereich (13, 14) bioaktive Substanzen umfasst.

9. Endoprothetisches Bauteil nach Anspruch 8, **dadurch gekennzeichnet, dass** die bioaktiven Substanzen einem Material beigemischt sind, aus dem der erste und/oder der zweite Bereich (13, 14) gefertigt ist oder, dass die bioaktiven Substanzen Schicht- oder bereichsweise in dem Material enthalten sind.

10. Endoprothetisches Bauteil nach Anspruch 8, **dadurch gekennzeichnet, dass** der erste und/oder der zweite Bereich (13, 14) mit der bioaktiven Substanz zumindest bereichsweise beschichtet ist.

11. Endoprothetisches Bauteil nach Anspruch 8, **dadurch gekennzeichnet, dass** die bioaktiven Substanzen in den Poren des zweiten Bereichs (14) angeordnet sind.

12. Endoprothetisches Bauteil nach einem der Ansprüche 8 bis 11, **dadurch gekennzeichnet, dass** die bioaktive Substanz Hydroxylapatit (HA) oder Tricalciumphosphat (TCP) oder Bioglas, insbesondere ein Bioglas, das als Hauptbestandteile SiO₂ (Siliziumdioxid), CaO (Calciumoxid), Na₂O (Natriumhyperoxid) und P₂O₅ (Phosphorpentoxid) aufweist, umfasst.

13. Endoprothetisches Bauteil nach einem der vorstehenden Ansprüche, **dadurch gekennzeichnet, dass** das Bauteil ein Wirbelsäulen-Implantat ist.

## Claims

1. Endoprosthetic component, the component comprising a first outer region (13) and a second inner region (14),
the material of the first region (13) having a greater strength than the material of the second region (14),
the first outer region of the component partially enclosing the second inner region, the first region (13) being designed to penetrate into adjacent bone material (11, 12) in a controlled manner when in the implanted state,
**characterized in that** the second region (14) has pores which have pore sizes of between 100 and 1000 µm, the porosity of the second region (14) being interconnected,
and at least part of the surface of the first region (13) projecting beyond the surface of the second region (14).

2. Endoprosthetic component according to claim 1, **characterized in that**
the first region (13) is designed to penetrate so deeply into the adjacent bone material (11, 12) that at least part of the surface of the second region (14) is in contact with the bone material (11, 12), such that a force transmission between bone material (11, 12) and the surface of the second region (14) takes place.

3. Endoprosthetic component according to either of the preceding claims, **characterized in that** the first region (13) is movably connected to the second region (14).

4. Endoprosthetic component according to any of the preceding claims, **characterized in that** the first region (13) is non-porous, i.e. preferably has a porosity of less than 5 vol.%.

5. Endoprosthetic component according to any of the preceding claims, **characterized in that** the second region (14) is porous, the porosity being between 75 and 85 vol.%.

6. Endoprosthetic component according to the preceding claim, **characterized in that** the second region (14) has pores which have pore sizes of between 400 and 600 µm.

7. Endoprosthetic component according to any of the preceding claims, **characterized in that** the first and/or the second region (13, 14) comprises ceramic material.

8. Endoprosthetic component according to any of the preceding claims, **characterized in that** the first and/or the second region (13, 14) comprises bioactive substances.

9. Endoprosthetic component according to claim 8, **characterized in that** the bioactive substances are admixed with a material from which the first and/or the second region (13, 14) is made, or **in that** the bioactive substances are contained in the material in layers or regions.

10. Endoprosthetic component according to claim 8, **characterized in that** the first and/or the second region (13, 14) is at least partially coated with the bioactive substance.

11. Endoprosthetic component according to claim 8, **characterized in that** the bioactive substances are arranged in the pores of the second region (14).

12. Endoprosthetic component according to any of claims 8 to 11, **characterized in that** the bioactive substance comprises hydroxyapatite (HA) or tricalcium phosphate (TCP) or bioglass, in particular a bioglass which has as main constituents SiO₂ (silicon dioxide), CaO (calcium oxide), Na₂O (sodium superoxide) and P₂O₅ (phosphorus pentoxide).

13. Endoprosthetic component according to any of the preceding claims, **characterized in that** the component is a vertebral column implant.

## Revendications

1. Composant endoprothétique, le composant comprenant une première région externe (13) et une seconde région interne (14), le matériau de la première région (13) présentant une résistance supérieure à celle du matériau de la seconde région (14), la première région externe du composant entourant partiellement la seconde région interne, la première région (13) étant conçue pour, à l'état implanté, pénétrer de manière contrôlée dans un matériau osseux (11, 12) adjacent, **caractérisé en ce que** la seconde région (14) comportant des pores dont les tailles sont comprises entre 100 et 1000 µm, la porosité de la seconde région (14) étant interconnectée, et au moins une partie de la surface de la première région (13) faisant saillie au-delà de la surface de la seconde région (14).

2. Composant endoprothétique selon la revendication 1, **caractérisé en ce que** la première région (13) est conçue pour pénétrer si profondément dans le matériau osseux (11, 12) adjacent qu'au moins une partie de la surface de la seconde région (14) est en contact avec le matériau osseux (11, 12), de sorte qu'une transmission de force se produise entre le matériau osseux (11, 12) et la surface de la seconde région (14).

3. Composant endoprothétique selon l'une des revendications précédentes, **caractérisé en ce que** la première région (13) est reliée de manière mobile à la seconde région (14).

4. Composant endoprothétique selon l'une des revendications précédentes, **caractérisé en ce que** la première région (13) n'est pas poreuse, c'est-à-dire qu'elle présente de préférence une porosité inférieure à 5 % en volume.

5. Composant endoprothétique selon l'une des revendications précédentes, **caractérisé en ce que** la seconde région (14) est poreuse, la porosité étant comprise entre 75 et 85 % en volume.

6. Composant endoprothétique selon la revendication précédente, **caractérisé en ce que** la seconde région (14) présente des pores dont la taille est comprise entre 400 et 600 µm.

7. Composant endoprothétique selon l'une des revendications précédentes, **caractérisé en ce que** la première et/ou la seconde région (13, 14) comprend un matériau céramique.

8. Composant endoprothétique selon l'une des revendications précédentes, **caractérisé en ce que** la première et/ou la seconde région (13, 14) comprend des substances bioactives.

9. Composant endoprothétique selon la revendication 8, **caractérisé en ce que** les substances bioactives sont mélangées à un matériau à partir duquel la première et/ou la seconde région (13, 14) est formée, ou **en ce que** les substances bioactives sont présentes dans le matériau en couches ou régions.

10. Composant endoprothétique selon la revendication 8, **caractérisé en ce que** la première et/ou la seconde région (13, 14) est revêtue de la substance bioactive au moins par régions.

11. Composant endoprothétique selon la revendication 8, **caractérisé en ce que** les substances bioactives sont disposées dans les pores de la seconde région (14).

12. Composant endoprothétique selon l'une des revendications 8 à 11, **caractérisé en ce que** la substance bioactive comprend de l'hydroxyapatite (HA) ou du phosphate tricalcique (TCP) ou du bioverre, en particulier un bioverre qui a comme constituants principaux SiO₂ (dioxyde de silicium), CaO (oxyde de calcium), Na₂O (hyperoxyde de sodium) et P₂O₅ (pentoxyde de phosphore).

13. Composant endoprothétique selon l'une des revendications précédentes, **caractérisé en ce que** le composant est un implant pour colonne vertébrale.
